# EUROPEAN PATENT APPLICATION

(11) **EP 1 130 031 A1**
(43) Date of publication of application: **05.09.2001**
(21) Application number: 00200664.1
(22) Date of filing: 25.02.2000
(51) Int. Cl.: C07K 14/78, A61P 9/00, A61K 38/39

(54) **Method for inhibiting angiogenesis using molecules that enhance plasmin formation or prolong plasmin activity**

(71) Applicant: Universitair Medisch Centrum Utrecht, 3584 CX Utrecht (NL)
(72) Inventor: Gebbink, Martijn Frans Ben Gerard, 3755 HL Eemnes (NL); Voest, Emile Eugene, 3768 AL Soest (NL)
(74) Representative: Ottevangers, Sietse Ulbe

(57) **Abstract**

Angiogenesis, the generation of new blood vessels, is a complex process that is controlled by a multitude of factors such as the extracellular matrix, components of the blood clotting system and angiogenic factors. The formation of blood vessels is accompanied by the formation of a so-called provisional matrix. Endothelial cells use the provisional matrix, among others, as a substrate for adhesion, migration and invasion. The provisional matrix is continuously build up and broken down until the new vessel is properly formed. This remodeling of the provisional matrix is regulated by the balanced action of various proteins. A key protein in the remodeling of the provisional matrix is the serine protease plasmin. The present invention provides means and methods based on proteinaceous molecules that enhance or sustain levels of plasmin near or at the site of unwanted angiogenesis through activation off plasminogen through tissue plasminogen activator. Through the means and methods of the invention the remodeling process of the provisional matrix can be tailored toward specific needs.

## Description

The present invention relates to methods and compositions for interfering in angiogenesis in a mammalian, in particular a human. More specifically, the present invention relates to novel methods and means for preventing or at least inhibiting angiogenesis by providing compounds which enhance or sustain the formation of plasmin.
The generation of new blood vessels, called angiogenesis or neovascularization, is essential for tissue growth and tissue repair (Folkman, 1995a; Folkman, 1996; Ossowski and Reich, 1983)(Regulation of angiogenesis (1997)edited by I.D. Golberg & E.M. Rosen Publischer Brikhäuser Verlach Basel Switserland). Under normal physiological conditions, angiogenesis is observed during wound healing, fetal and embryonal development and formation of the corpus luteum, endometrium and placenta. Angiogenesis has also been found to play a role in diseases. Persistent, unwanted angiogenesis occurs in a multiplicity of disease states, including tumor growth, metastasis and diabetic retinopathy. In these disease states, prevention of angiogenesis could avert the damage caused by the invasion of new vessels. Strategies to prevent the development of new vessels in tumors and metastases have been effective in suppressing growth of these tumors (Folkman, 1995b; Voest, 1996). Therapies directed at control of the angiogenic process could lead to the abrogation or mitigation of diseases in which angiogenesis is involved.

Angiogenesis is a highly regulated proces. Angiogenesis is initiated by the release of angiogenic stimuli, such as vascular endothelial growth factor (VEGF). Such stimuli act on the endothelial cells, which line the lumen of blood vessels. Upon stimulation the endothelial cells mediate the degradation of the basement membrane, which surround the endothelial cells in normal vessels. Angiogenic stimuli induce the formation of a provisional matrix and induce migration, proliferation and invasion of endothelial cells into tissue to form a new vessel.

The formation of provisional matrix is a hallmark of angiogenesis. Endothelial cells use the provisional matrix as substrate for adhesion, migration and invasion. As such the provisional matrix is also essential for endothelial cell survival, i.e. provisional matrix proteins can protect endothelial cells from undergoing apoptosis (Isik et al., 1998). The provisional matrix is formed by the action of many molecules that also play a prominent role in coagulation and fibrinolysis. As such the formation of a provisional matrix resembles the formation and degradation of a blood clot or hemostatic plug. The formation of the provisional matrix is initiated by the action of tissue factor. Tissue factor is present in the subendothelial matrix on cancer cells (Hu et al., 1994) and induced on the cell surface of stimulated endothelial cells (Zucker et al., 1998). Expression of tissue factor has been linked to the angiogeneic properties of malignant tumors (Ruf and Mueller, 1996). As a result of tissue factor action thrombin is formed, which generates fibrin from fibrinogen. The provisional matrix contains many proteins, including vitronectin, that are produced in the liver and derived from blood. These proteins are recruted from blood, when vessels become permeable upon stimulation with angiogenic stimuli and are temporarily deposited to form part of the provisional matrix.

The provisional matrix is continuously generated and broken down, a process called remodeling, until a new functional vessel has been properly formed. Remodeling of the provisional matrix is strictly regulated by the balanced action of molecules, involved in the generation and in the degradation of the matrix. The formation of the serine protease plasmin through activation of its zymogen plasminogen is a key step in this process. Plasmin mediates proteolysis of the provisional matrix by cleaving fibrin, called fibrinolysis, as well as other matrix components. In addition plasmin mediates proteolysis indirectly by the activation of metalloproteinases, which in turn degrade other components of the extracellular matrix, including collagen. Given its pivotal role in matrix remodeling the formation of plasmin is tightly controlled by the balance between the action of plasminogen activators, plasminogen activator inhibitors and by inhibitors of plasmin, such as α2antiplasmin. A shift in this balance, by either increasing the levels or activity of inhibitors or by enhancing the formation of plasmin have been shown to have profound effects on either endothelial cell adhesion, migration, angiogenesis, metastasis or tumor growth.
The present invention now provides novel methods and means based on proteinaceous molecules that enhance or sustain levels of plasmin near or at the site of unwanted angiogenesis through activation of plasminogen through tissue plasminogen activator.
Efficient activation of plasminogen by tissue plasminogen activator (tPA), a serine protease expressed almost exclusively by stimulated endothelial cells (Mandriota and Pepper, 1997), requires the presence of a cofactor, and fibrin is regarded as the principal fibrinolytic stimulator, but other proteins of the extracellular matrix, such as collagens, may also enhance plasminogen activation (Stack et al., 1990). Binding and activation of plasminogen is mediated by carboxyterminal lysine residues that are generated in fibrin during plasmin digestion (Fleury et al., 1993). Removal of the carboxy-terminal lysine residues by carboxypeptidases abrogates the stimulatory effect of fibrin.
Thrombin-activatable fibrinolysis inhibitor (TAFI), also named plasma procarboxypeptidase B or procarboxypeptidase U is a physiological fibrinolysis inhibitor (Nesheim et al., 1997). Activition of TAFI is mediated by thrombin or plasmin and thrombin mediated activation of TAFI is greatly enhanced by thrombomodulin, a cell surface protein made almost exclusively by endothelial cells. Therefore TAFI is believed to be a modulator of the provisional matrix as it occurs during disease associated angiogenesis. In summary, the efficient formation of plasmin is mediated by proteins or protein fragments that contain an important carboxyterminal lysine residue. An important aspect of the present invention is to enhance or sustain the formation of plasmin, resulting in at least decreased amounts of provisional matrix and at least decreased ability of the provisional matrix to support angiogenesis. For example, proteinaceous molecules comprising a lysin and/or arginin residue and/or a functional equivalent thereof, capable of providing enhanced levels of plasmin in a mammalian through tPA mediated plasminogen activation may be used to stimulate the formation of plasmin. Alternatively and/or additionally, inhibitors of carboxypeptidases may be used to prevent the removal of carboxyterminal lysine residues that stimulate plasmin formation. This novel approach applies to a variety of angiogenesis mediated diseases.
Thus the present invention provides a proteinaceous molecule comprising a lysin and/or arginin residue and/or a functional equivalent thereof, capable of providing enhanced levels of plasmin in a mammalian through tPA mediated plasminogen activation. A functional equivalent of such a residue or molecule is a residue that is capable of performing the same function as the original residue, i.e. stimulate directly or indirectly the formation of plasmin. The stimulation is typically tPA dependent, therefore a tPA binding site is preferably present in the proteinaceous molecules according to the invention. A partial β-sheet may also be typically present in the proteinaceous molecules according to the invention. A good starting point for obtaining or designing proteinaceous molecules according to the invention are components of the extra cellular matrix, in particular fibrin and vitronectin, which are natural activators according to the invention. Functiqnal equivalents thereof can be prepared by people of skill in the art without needing further explanations here. For sake of ease of production it is preferred that the proteinaceous molecules according to the invention are about 15- 35 amin acid residues long. It is preferred that the lysin, arginin, or their functional equivalent is carboxy-terminal or becomes carboxy-terminal in *situ.*

Several anti-angiogenic compounds have been used to prevent angiogenesis. The most common approach to inhibit angiogenesis, metastasis or tumor growth is to competitively inhibit angiogenic proteins or molecules. Examples include molecules that block growth factor mediated induction of angiogenesis, such as neutralizing antibodies to vascular endothelial growth factor.(Kuiper et al., 1998). Several compounds have been described that inhibit angiogenesis by inhibiting proteases, such as plasminogen activator, plasmin or metalloproteinases. These compounds include plasminogen activator inhibitor 1, aprotinin, batimastat and marimastat. The mechanism of other anti-angiogenic compounds, including thalidomide, some of which may have unwanted side effects, is less well known.

The present invention discloses the use of a novel strategy to prevent angiogenesis. In the present invention molecules are used that function as cofactor in tPA mediated formation of plasmin from plasminogen. Upon treatment with such cofactors excess plasmin is being generated, resulting in enhanced proteolysis and detachment of cells. The strategy is non-competitive and the process very efficient, because a single molecule can catalyze the formation of many plasmin molecules. In addition, by generating excess plasmin, this novel approach offers, in contrast to conventional strategies aimed at inhibiting proteases, a unique, efficient way to destroy the micro-environment and shrinkage of the affected pathological tissue. Finally, the strategy is specific for cells that express tPA, i.e. activated endothelial cells.
One of the most prominent applications of the present invention lies in the treatment of diseases, including but not limited to cancer, in which degradation of a provisional matrix plays an essential role.

The use of the proteinaceous molecules according to the invention is thus apparent and also part of the present invention. Thus in one embodiment the invention provides the use of a proteinaceous molecule comprising a lysin and/or arginin residue and/or a functional equivalent thereof, capable of providing enhanced levels of plasmin in a mammalian through tPA mediated plasminogen activation in the preparation of a medicament for the treatment of diseases related with angiogenesis. In a further embodiment the invention provides the use of a proteinaceous molecule comprising a lysin and/or arginin residue and/or a functional equivalent thereof, capable of providing enhanced levels of plasmin in a mammalian through tPA mediated plasminogen activation in the preparation of a medicament for the prevention of unwanted angiogenesis.
In yet a further embodiment the invention provides the use of a proteinaceous molecule comprising a lysin and/or arginin residue and/or a functional equivalent thereof, capable of providing enhanced levels of plasmin in a mammalian through tPA mediated plasminogen activation in the preparation of a medicament for the breakdown of extracellular matrix components.

According to the invention it is also possible to provide the presence of molecules according to the invention in situ, by inhibiting their removal, e.g. through the presence of carboxypeptidase inhibitors. The combination of both is especially advantageous.

The invention also includes a method for the treatment of diseases associated with and/or dependent on angiogenesis comprising administering to a patient an effective amount of a proteinaceous molecule comprising a lysin and/or arginin residue.and/or a functional equivalent thereof, capable of providing enhanced levels of plasmin in a mammalian through tPA mediated plasminogen activation, also optionally together with a carboxypeptidase inhibitor.Optinally, both activities are pressed in one (fusion) molecule.
One example of a disease mediated by angiogenesis is ocular neovascular disease. This disease is characterized by invasion of new blood vessels into the structures of the eye, such as the retina or cornea. It is the most common cause of blindness and is involved in approximately twenty eye diseases. In age-related macular degeneration, the associated visual problems are caused by an ingrowth of choriodal capillaries through defects in Bruch's membrane with proliferation of fibrovascular tissue beneath the retinal pigment epithelium. Angiogenic damage is also associated with diabetic retinopathy, retinopathy of prematurity, corneal graft rejection, neovascular glaucoma and retrolental fibroplasia. Other diseases associated with neovascularization include, but are not limited to, epidemic keratoconjunctivitis, Vitamin A deficiency, contact lens overwear, atopic keratitis, superior limbic keratitis, pterygium keratitis sicca, sjogrens, acne rosacea, phylectenulosis, syphilis, Mycobacteria infections, lipid degeneration, chemical burns, bacterial ulcers, fungal ulcers, Herpes simplex infections, Herpes zoster infections, protozoan infections, Kaposi sarcoma, Mooren ulcer, Terrien's mariginal degeneration, marginal keratolysis, rheumatoid arthritis, systemic lupus, polyarteritis, trauma, Wegeners sarcoidosis, Scleritis, Steven's Johnson disease, periphigoid radial keratotomy, and corneal graph rejection.

Diseases associated with retinal/choroidal neovascularization include, but are not limited to, diabetic retinopathy, macular degeneration, sickle cell anemia, sarcoid, syphilis, pseudoxanthoma elasticum, Pagets disease, vein occlusion, artery occlusion, carotid obstructive disease, chronic uveitis/vitritis, mycobacterial infections, Lyme's disease, systemic lupus erythematosis, retinopathy of prematurity, Eales disease, Bechets disease, infections causing a retinis or choroiditis, presumed ocular histoplasmosis, Bests disease, myopia, optic pits, Stargarts disease, pars planitis, chronic retinal detachment, hyperviscosity syndromes, toxoplasmosis, trauma and post-laser complications. Other diseases include, but are not limited to, diseases associated with rubeosis (neovacularization of the angle) and diseases caused by the abnormal proliferation of fibrovascular or fibrous tissue including all forms of proliferative vitreoretinopathy. Another disease in which angiogenesis is believed to be involved is rheumatoid arthritis. The blood vessels in the synovial lining of the joints undergo angiogenesis. In addition to forming new vascular networks, the endothelial cells release factors and reactive oxygen species that lead to pannus growth and cartilage destruction. The factors involved in angiogenesis may actively contribute to, and help maintain, the chronically inflamed state of rhaumatoid arthritis.

Factors associated with angiogenesis may also have a role in osteoarthritis. The activation of the chondrocytes by angiogenic-related factors contibutes to the destruction of the joint. At a later stage, the angiogenic factors would promote new bone formation. Therapeutic intervention that prevents the bone destruction could halt the progress of the disease and provide relief for persons suffering with arthritis. Chronic inflammation may also involve pathological angiogenesis. Such disease states as ulcerative colitis and Crohn's disease show histological changes with the ingrowth of new blood vessels into the inflamed tissues. Bartonellosis, a bacterial infection found in South America, can result in a chronic stage that is characterized by proliferation of vascular endothelial cells. Another pathological role associated with angiogenesis is found in atherosclerosis. The plaques formed within the lumen of blood vessels have been shown to have angiogenic stimulatory activity.

One of the most frequent angiogeneic disease of childhood is the hemangioma. In most cases, the tumors are benign and regress without intervention. In more severe cases, the tumors progress to large cavernous and infiltrative forms and create clinical complications. Systemic forms of hemangiomas, the hemangiomatoses, have a high mortality rate. Therapy-resistant hemangiomas exist. Angiogenesis is also responsible for damage found in hereditary hemorrhagic telangiectasia. This is an inherited disease characterized by multiple small angiomas, tumors of blood or lymph vessels. The angiomas are found in the skin and mucous membranes, often accompanied by epistaxix (nosebleeds) or gastrointestinal bleeding and sometimes with pulmonary or hepatic arteriovenous fistula.

Angiogenesis is prominent in solid tumor formation and metastasis. Angiogenic factors have been found associated with several solid tumors. Tumors in which angiogenesis is involved include, but are not limited to, solid tumors and benign tumors, such as neurofibroma, trachoma and pyogenic granulomas. Prevention of angiogenesis could halt the growth of these tumors and the resultant damage.

Angiogenesis has been associated with blood-born tumors, such as leukemias, any of the various acute or chronic neoplastic diseases of the bone marrow in which unrestrained proliferation of white blood cells occurs, usually accompanied by anemia, impaired blood clotting, and enlargement of the lymph nodes, liver and spleen. It is believed that angiogenesis plays a role in the abnormalities in the bone marrow that give rise to leukemia-like tumors.

Angiogenesis is important in tumor metastasis. Angiogenesis allows tumor cells to enter the blood stream and to settle into a secondary site. Therefore, prevention of angiogenesis could lead to the prevention of metastasis.

The present invention provides among other things a method for the treatment of angiogenesis dependent or related diseases by using molecules that efficiently promote or sustain tPA mediated plasmin formation. A major advantage for therapeutical application is that the method is selective for tPA. This implies that molecules based on the invention have a restricted and localized action, namely at sites were tPA is available, such as on the surface of activated endothelial cells or certain tumor cells. In addition, the method offers the advantage that through enhanced plasmin formation, rather than decreased plasmin formation, the pathological tissue may decline.

Based on this invention, many different molecules may be designed. Molecules may be derived from proteins present in nature but may also be generated completely artificially as long as they contain a lysine, an arginine, an analogous moiety or a modified form of these, and as long as they stimulate tPA mediated plasmin formation. In an alternative embodiment of the invention the molecule is a natural plasmin generated cleavage product derived from fibrin. Non-limiting examples of other molecules based on the invention are plasmin generated cleavage products of other provisional matrix proteins, including, but not limited to vitronectin. On the other hand however, also molecules may be used that are artificially made.

It is clear to a person skilled in the art that only the essential part or parts of a protein are required in the molecules of the invention. Thus deletions/insertions or mutations in non-relevant parts of the protein are anticipated to be equally or more effective as the entire molecule.
It is also clear to a person skilled in the art that the protein molecule of the invention may contain further functional units derived from different molecules exisiting in nature or artificial to broaden the functionality of the molecule of invention.
It is also clear to a person skilled in the art that a molecule artificially made, structurally related to the molecule of the invention but not containing a protein moiety are anticipated to be equally or more effective.

Upon further study of the specification, drawings and appended claims, objects and advantages of this invention will become apparent to those skilled in the art.

The invention is illustrated by the following drawings and examples, which are not to be construed in any way as imposing limitations upon the scope therof. On the contrary, it is to be clearly understood that resort may be had to various other embodiments, modifications, and equivalents thereof which, after reading the description herein, may suggest themselves to those skilled in the art without departing from the spirit of the present invention and/or the scope of the appended claims. To illustrate the method we have used fibrin degradation products (FDP) as molecules that are capable of stimulating the tPA mediated formation of plasmin. In that respect, FDP serve as a model for a broad variety of molecules.

### EXAMPLES

### EXAMPLE 1 : Effect of FDP on subcutaneous tumor growth.

A model of subcutaneous tumor growth of a mouse C26 colon carcinoma was used to evaluate the effects of FDP. Eleven days after tumor cell inoculation tumors of the control group had reached a volume of 2719 ± 366 mm³. In mice treated with FDP the mean tumor growth was 719 ± 188 mm³. At similar concentrations, endostatin, another molecule with the ability to stimulate tPA mediated plasmin formation (see below), suppressed tumor growth as well but was slightly less effectively (tumor volume 1112 ± *372 mm*^{*3*}). Treatment with tPA, which generates plasmin, suppressed tumor growth to a similar degree (tumor volume 492 ± 215 mm³) as FDP. These result demonstrate that two molecules which fullfil the criteria of the proposed invention potently inhibited tumor growth.

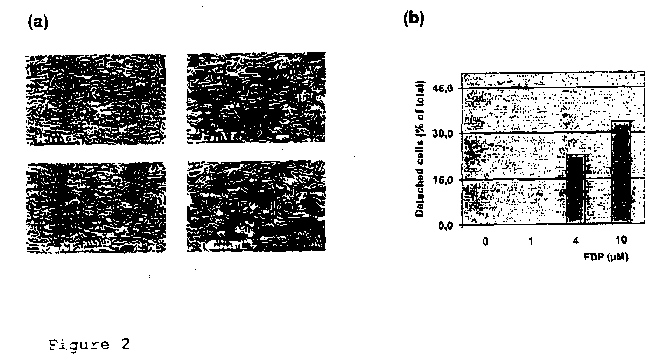

EXAMPLE 2 : Effect of FDP on endothelial cells in vitro. Treatment of BPAEC cells grown in monolayers caused dramatic morphological changes within 24 hr, while untreated cells retain their characteristic cell shape (Fig 2a). More than 30% of the cells treated with FDP detached from the substratum (Fig 2b).

EXAMPLE 3 : Effect of FDP, FPD derived synthetic peptide (aa 148-160), fragment (aa. 262-367) vitronectin and endostatin on plasmin generation. Various concentrations were added.

### Materials and Methods

### Endostatin

The cDNA for murine endostatin (kindly provided by dr. Fukai, Boston) was amplified by PCR and cloned into the prokaryotic expression vector pET15b. Recombinant murine endostatin was produced by *Escherichia coli* and purified on Ni²⁺-NTA-beads (Qiagen) as described (Boehm et al., 1997; O'Reilly et al., 1997).

### Fibrin Degradation Products (FDP)

Fibrin degradation products (FDP) were generated by plasmin digestion of fibrin. Human fibrinogen (Sigma, The Netherlands) at a concentration of 5 mg/ml was allowed to clot in 25 mM Tris-HCl pH 7.4, 150 mM NaCl by the addition of thrombin (1.32 µM final concentration) for 3 hr at 37 °C (thrombin was a generous gift of Dr. W. Kisiel, University of New Mexico, Albequerque, NM, USA). Clot lysis was accomplished by addition of plasmin (Roche, ). Plasmin was added at a molar ratio (plasmin:fibrinogen) of 1:300. Lysis was performed for 20 hr at room temperature. After centrifugation the supernatant was passed through an aprotinin-sepharose column to remove plasmin. FDP were stored at -20 °C. Activity was determined using a plasminogen activation assay.

### Vitronectin fragments

The cDNA for human vitronectin was cloned from human liver, amplified by PCR and cloned into the prokaryotic expression vector pET15b. Recombinant vitronectin protein was produced by *Escherichia coli* as described for endostatin.

### Measurement of plasmin activity

Reactions were performed at 37 °C in HBS buffer (20 mM Hepes, 4 mM KCl, 137 mM NaCl, 3 mM CaCl₂, 0.1 % BSA, pH 7.4) containing 50 µg/ml plasminogen with or without the addition of endostatin or FDP. The reactions were started by the addition tPA at a final concentration of 30 U/ml. At several time points, 20 µl samples were taken and stopped with 20 µl buffer containing 150 mM εACA and 150 mM EDTA. Plasmin activity was determined in 96-well plates after the addition of 20 µl chromogenic substrate S-2251 at a final concentration of 1.6 mM. Increase in absorbance was measured at 405 nm for 10 min.

### Cells and culture conditions

The mouse colon adenocarcinoma cell line C-26 was maintained as a monolayer culture in Dulbecco's Minimal Essential Medium (DMEM) supplemented with 10% heat-inactivated fetal calf serum, penicillin (100 units/ml), and streptomycin (100 µ g/ml) in a 5% CO₂ environment. Confluent cultures were harvested by brief trypsinization (0.05 trypsin in 0.02% EDTA), washed 3 times with PBS, and resuspended to a final concentration of 5x10⁶ cells/ml. The presence of single cell suspension was confirmed by phase contrast microscopy, and cell viability was determined by trypan blue staining.

### Cell detachment assay

BPAEC (CCL-209) was obtained from the American Type Culture Collection (Rockville, MD) BPAEC were grown in Dulbecco's Modified Essential Medium (DMEM) with 20% fetal calf serum (FCS) and antibodies. BPAEC were seeded in 24-well culture dishes and solvent control in DMEM containing 20 mM Hepes and 10 % FCS. Cell morphologh was examined by phase contrast microscopy. After 48 to 72 hours the detached cells were removed and the remaining attached cells were removed by trypsin exposure and counted. The percentage of detached cells was calculated.

### Mice

Male BALB/c mice were purchased from the General Animal Laboratory, University Medical Centre (Utrecht, The Netherlands). Animals were maintained with food and water ad libitum and kept on a 12-hour light/12 hour dark cycle. All animal studies were conducted on male, 6 to 8 week old mice.

### Tumor experiments

Male BALB/c mice were inoculated with 10⁶ C26 colon carcinoma cells. Mice were treated daily by subcutanous injection with either saline or the compound to be tested for a period of approximately 14 days. Serial caliper measurements of perpendicular diameters were used to calculate tumor volumes in mm³ using the formula: longest diameter x shortest diameter² x 0.52.

### Statistical Analysis

The statistical significance of differences between groups was calculated by applying Students 2-tailed t-test. Results are presented as the mean ± standard error of the mean.

### Figure Legend

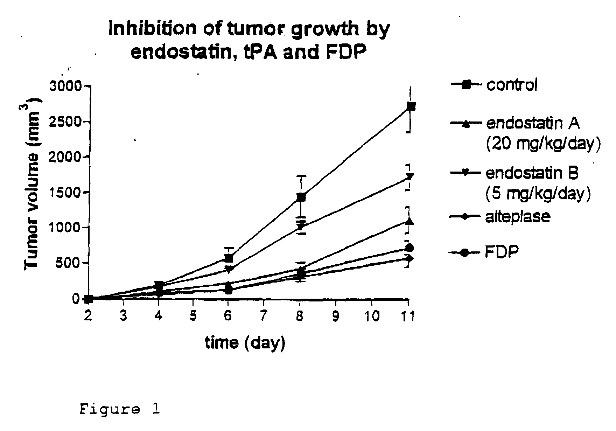

Figure 1 : Effect of FDP on tumor growth. For comparison, treatments with Alteplase® (recombinant tPA) or endostatin is shown. FDP was given continuously using Alzet® pumps (Alza, Palo Alto, CA, USA, type 2001 or 2002) loaded with 200 µl FDP (5 mg/ml). In addition every other day FDP was given subcutaneously at a dose of 7 mg/kg.

Figure 2 : Effect of FDP on endothelial cell attachment. (a) Micrographs showing BPAEC monolayers of (I) control cells treated with PBS for 24 hr; (II) cells treated with 1 µM FDP; (III) cells treated with 4 µM FDP; cells treated with 10 µM FDP.

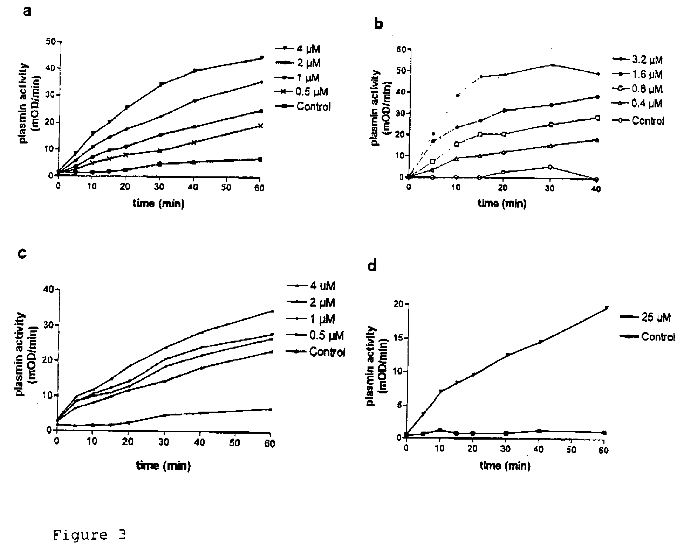

Figure 3 : Effect on plasmin activity of endostatin (a), vitronectin fragment + (aa. 262-367) (b) FDP (c), peptide derived from FDP (aa. 148-160) (d)

### Reference List

Boehm,T., Folkman,J., Browder,T., and O'Reilly,M.S. (1997). Antiangiogenic therapy of experimental cancer does not induce acquired drug resistance. Nature *390,* 404-407.

Fleury,V., Loyau,S., Lijnen,H.R., Nieuwenhuizen,W., and Angles-Cano,E. (1993). Molecular assembly of plasminogen and tissue-type plasminogen activator on an evolving fibrin surface. Eur. J. Biochem. *216,* 549-556.

Folkman,J. (1995a). Angiogenesis in cancer, vascular, rheumatoid and other disease. Nat. Med. *1*, 27-31.

Folkman,J. (1995b). Clinical applications of research on angiogenesis. Semin. Med. Beth Israel Hosp. 333, 1757-1763.

Folkman,J. (1996). Fighting cancer by attacking its blood supply. Sci. Am. *275,* 150-154.

Ge,M., Tang, G., Ryan,T.J., and Malik, A.B. (1992). Fibrinogen degradation product fragment D induces endothelial cell detachment by activation of cell-mediated fibrinolysis. J. Clin. Invest 90, 2508-2516.

Hu,T., Bach,R.R., Horton,R., Konigsberg,W.H., and Todd,M.B. (1994). Procoagulant activity in cancer cells is dependent on tissue factor expression. Oncol. Res 6, 321-327.

Isik,F.F., Gibran,N.S., Jang,Y.C., Sandell,L., and Schwartz,S.M. (1998). Vitronectin decreases microvascular endothelial cell apoptosis. J Cell Physiol. *175,* 149-155.

Kuiper, R.A., Schellekens, J.H., Blijham, G.H. , Beyeren, J.H. and Voest, E.E. (1998). Clinical research on antianjiogenic therapy. Pharmacol. REs. 37(1), 1-16.

Mandriota,S.J. and Pepper,M.S. (1997). Vascular endothelial growth factor-induced in vitro angiogenesis and plasminogen activator expression are dependent on endogenous basic fibroblast growth factor. J. Cell Sci. *110,* 2293-2302.

Nesheim,M., Wang,W., Boffa,M., Nagashima,M., Morser,J., and Bajzar,L. (1997). Thrombin, thrombomodulin and TAFI in the molecular link between coagulation and fibrinolysis. Thromb. Haemost. 78, 386-391.

O'Reilly, M.S., Boehm,T., Shing,Y., Fukai,N., Vasios,G., Lane,W.S., Flynn,E., Birkhead,J.R., Olsen,B.R. , and Folkman,J. (1997). Endostatin: an endogenous inhibitor of angiogenesis and tumor growth. Cell *88*, 277-285.

Ossowski,L.K. and Reich,E. (1983). Antibodies to plasminogen activator inhibit human tumor metastasis. Cell 35, 611-619.

Ruf,W. and Mueller,B.M. (1996). Tissue factor in cancer angiogenesis and metastasis. Curr. Opin. Hematol. 3, 379-384.

Stack,S., Gonzalez-Gronow,M., and Pizzo,S.V. (1990). Regulation of plasminogen activation by components of the extracellular matrix. Biochemistry 29, 4966-4970.

Voest,E.E. (1996). Inhibitors of angiogenesis in a clinical perspective. Anticancer Drugs 7, 727.

Zucker,S., Mirza,H., Conner,C.E., Lorenz,A.F., Drews,M.H., Bahou,W.F., and Jesty,J. (1998). Vascular endothelial growth factor induces tissue factor and matrix metalloproteinase production in endothelial cells: conversion of prothrombin to thrombin results in progelatinase A activation and cell proliferation. Int. J Cancer 75, 780-786

## Claims

1. A proteinaceous molecule comprising a lysin and/or arginin residue and/or a functional equivalent thereof, capable of providing enhanced levels of plasmin in a mammalian through tPA mediated plasminogen activation for use as a pharmaceutical.

2. Use of a proteinaceous molecule comprising a lysin and/or arginin residue and/or a functional equivalent thereof, capable of providing enhanced levels of plasmin in a mammalian through tPA mediated plasminogen activation in the preparation of a medicament for the treatment of diseases related with angiogenesis.

3. Use of a proteinaceous molecule comprising a lysin and/or arginin residue and/or a functional equivalent thereof, capable of providing enhanced levels of plasmin in a mammalian through tPA mediated plasminogen activation in the preparation of a medicament for the prevention of unwanted angiogenesis.

4. Use of a proteinaceous molecule comprising a lysin and/or arginin residue and/or a functional equivalent thereof, capable of providing enhanced levels of plasmin in a mammalian through tPA mediated plasminogen activation in the preparation of a medicament for the breakdown of extracellular matrix components.

5. Use according to any one of claims 1-4, wherein said proteinaceous molecule is derived from fibrin, vitronectin, or another component of the extracellular matrix.

6. Use according to claim 5, wherein said proteinaceous molecule is derivable by proteolytic cleavage from an extracellular matrix component.

7. Use according to any one of claims 1-6 , wherein said lysin, arginin or functional equivalent thereof is a residue at or near a carboxy-terminus of said proteinaceous molecule.

8. Use according to any one of claims 1-7, which comprises from 15-30 amino acid residues.

9. Use according to any one of claims 1-8, wherein said proteinaceous molecule comprises a β-sheet and/or a tPA binding site.

10. Use according to any one of claims 1-8, in combination with an inhibitor of carboxypeptidase.

11. A method for the treatment of diseases associated with and/or dependent on angiogenesis comprising administering to a patient an effective amount of a proteinaceous molecule comprising a lysin and/or arginin residue and/or a functional equivalent thereof, capable of providing enhanced levels of plasmin in a mammalian through tPA mediated plasminogen activation.

12. A method according to claim 11, further comprising administering a carboxypeptidase inhibitor.

13. A method according to claim 11 or 12, in which the disease is associated with undesired angiogenesis.

14. A method according to claim 11, 12 or 13, wherein extracellular matrix components at the site of unwanted angiogenesis are degraded upon administration.

15. A pharmaceutical formulation comprising a proteinaceous molecule as defined in any one of claims 1-14.

16. A pharmaceutical formulation according to claim 15 further comprising a carboxypeptidase inhibitor.

17. A peptide of about 10-150 amino acid residues, comprising the sequence 262-367 of vitronectin, or a functional fragment and/or derivative thereof.

18. A peptide according to claim 17, comprising 95-110 residues.
